(19) Europäisches Patentamt
European Patent Office
Office européen des brevets

(11) EP 4 417 646 A1

(12) EUROPEAN PATENT APPLICATION
published in accordance with Art. 153(4) EPC

(43) Date of publication:
**21.08.2024 Bulletin 2024/34**

(21) Application number: **23831786.1**

(22) Date of filing: **19.06.2023**

(51) International Patent Classification (IPC):
***C08L 5/08*** (2006.01) ***C08L 1/28*** (2006.01)
***C08L 3/04*** (2006.01) ***C08B 37/08*** (2006.01)
***C08B 31/04*** (2006.01) ***C08B 11/12*** (2006.01)
***C08J 3/24*** (2006.01)

(52) Cooperative Patent Classification (CPC):
**C08B 11/12; C08B 31/04; C08B 37/003; C08J 3/24; C08L 1/28; C08L 3/04; C08L 5/08**

(86) International application number:
**PCT/KR2023/008444**

(87) International publication number:
**WO 2024/005426 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.06.2022 KR 20220078925**
**15.06.2023 KR 20230076830**

(71) Applicant: **LG Chem, Ltd.**
**Yeongdeungpo-gu**
**Seoul 07336 (KR)**

(72) Inventors:
- **CHO, Beomshin**
**Daejeon 34122 (KR)**
- **KANG, Sunah**
**Daejeon 34122 (KR)**
- **YUN, Haesung**
**Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

# (54) BIODEGRADABLE SUPER ABSORBENT POLYMER AND PREPARATION METHOD THEREOF

(57) The present disclosure relates to a super absorbent polymer exhibiting excellent biodegradability without deterioration in physical properties of the super absorbent polymer, and a preparation method of the same.

【FIG. 1】

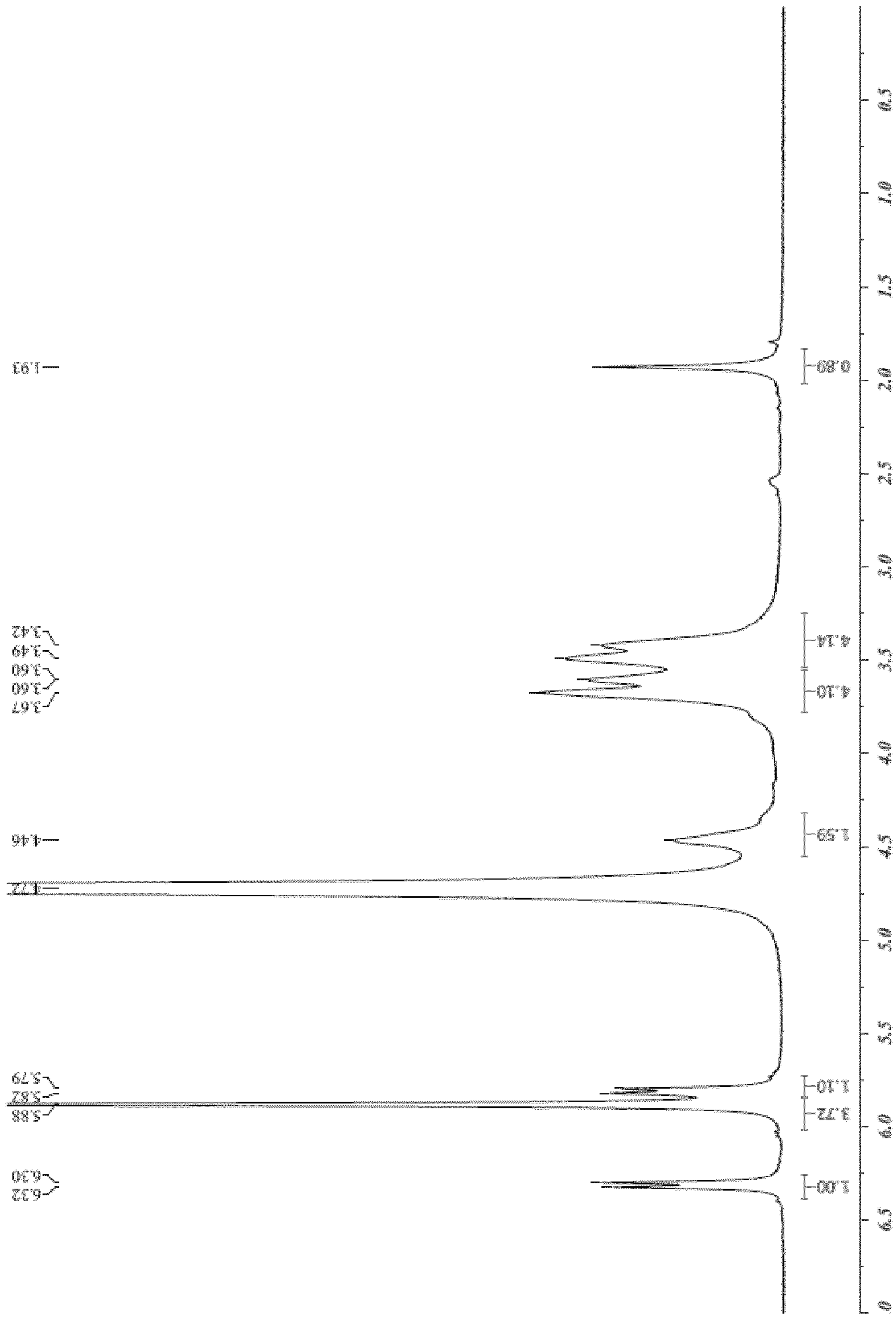
1.93
3.42
3.49
3.60
3.60
3.67
4.46
4.72
5.79
5.82
5.88
6.30
6.32
0.89
4.14
4.10
1.59
1.10
3.72
1.00
0.5
1.0
1.5
2.0
2.5
3.0
3.5
4.0
4.5
5.0
5.5
6.0
6.5

## Description

[TECHNICAL FIELD]

Cross-reference to Related Application(s)

This application claims the benefits of Korean Patent Applications No. 10-2022-0078925 filed on June 28, 2022 and No. 10-2023-0076830 filed on June 15, 2023 with the Korean Intellectual Property Office, the disclosure of which is incorporated herein by reference in its entirety.

The present disclosure relates to a biodegradable super absorbent polymer exhibiting excellent biodegradability without deterioration in physical properties of the super absorbent polymer, and a preparation method of the same.

[BACKGROUND OF ART]

A super absorbent polymer (SAP) is a type of synthetic polymeric material capable of absorbing 500 to 1000 times its own weight of moisture. Various manufacturers have denominated it with different names, such as SAM (Super Absorbency Material), AGM (Absorbent Gel Material), and the like. Such super absorbent polymers started to be practically applied in sanitary products, and they are now being widely used not only for hygiene products such as disposable diapers for children, etc., but also for water retaining soil products for gardening, water stop materials for the civil engineering and construction, sheets for raising seedling, fresh-keeping agents for food distribution fields, materials for poultices, or in the field of electrical insulation.

Such a super absorbent polymer is typically produced after a hydrogel polymer is obtained by subjecting an acrylic acid-based monomer to bulk polymerization or suspension polymerization together with a cross-linking agent in the presence of a polymerization initiator. Therefore, most conventional super absorbent polymers have little biodegradability, which causes environmental problems when performing waste disposal. Specifically, when several products to which super absorbent polymer is applied are buried and disposed, such super absorbent polymer is not decomposed by bacteria or microorganisms in the ground, which may cause environmental pollution.

In view of the above, attempts have been made on super absorbent polymers that exhibit excellent biodegradability using biomass-derived materials, but it has not been easy to produce a biodegradable super absorbent polymer that can be economically produced while exhibiting various physical properties comparable to those of conventional super absorbent polymers.

Accordingly, there is a continuous demand for the development of a super absorbent polymer-related technology capable of exhibiting biodegradability without deteriorating basic physical properties of the super absorbent polymer.

[DETAILED DESCRIPTION OF THE INVENTION]

[Technical Problem]

Accordingly, there are provided a super absorbent polymer exhibiting excellent biodegradability without deterioration in physical properties of the super absorbent polymer, and a preparation method of the same.

[Technical Solution]

According to an embodiment of the present disclosure, there is provided a biodegradable super absorbent polymer including a cross-linked polymer of a monomer containing a modified polysaccharide having at least one of a maleic acid group (-OCOCH=CHCOOH) and a carboxymethyl group (-$CH_2$-COOH),

wherein at least a part of the maleic acid group and the carboxymethyl group is neutralized,
the cross-linked polymer contains 60 parts by weight or more of a modified polysaccharide-derived repeating unit, and a molecular weight of the modified polysaccharide is 190,000 g/mol or more.

According to another embodiment of the present disclosure, there is provided a preparation method of a biodegradable super absorbent polymer including the steps of:

preparing a monomer composition including a modified polysaccharide having at least one of a maleic acid group (-OCOCH=CHCOOH) and a carboxymethyl group (-$CH_2$-COOH) in the presence of a polymerization initiator;
preparing a hydrogel polymer by performing cross-linking polymerization on the monomer composition; and
drying, pulverizing, and classifying the hydrogel polymer,

wherein at least a part of the maleic acid group and the carboxymethyl group is neutralized,
the cross-linked polymer contains 60 parts by weight or more of a modified polysaccharide-derived repeating unit, and a molecular weight of the modified polysaccharide is 190,000 g/mol or more.

Further, according to another embodiment of the present disclosure, there is provided a hygiene product including the biodegradable super absorbent polymer.

[ADVANTAGEOUS EFFECTS]

The biodegradable super absorbent polymer according to the present disclosure exhibits excellent biodegradability by using a chemically modified polysaccharide. Also, the biodegradable super absorbent polymer of the present disclosure uses a modified polysaccharide having a weight average molecular weight of a certain level or higher, and thus can be subjected to polymerization and cross-linking without unreacted modified polysaccharide, so that general physical properties of the super absorbent polymer may not be deteriorated. Therefore, the biodegradable super absorbent polymer may not cause a problem of environmental pollution at the time of disposal even if it is applied to various hygiene products.

[BRIEF DESCRIPTION OF THE DRAWINGS]

FIG. 1 shows a $^1H$ NMR spectrum of maleated chitosan prepared in Preparation Example 1.
FIG. 2 shows a $^1H$ NMR spectrum of maleated modified starch prepared in Preparation Example 2.
FIG. 3 shows a $^1H$ NMR spectrum of carboxymethylated modified starch prepared in Preparation Example 3.
FIG. 4 shows a $^1H$ NMR spectrum of carboxymethylated modified starch prepared in Preparation Example 4.
FIG. 5 shows a $^1H$ NMR spectrum of carboxymethylated modified starch prepared in Preparation Example 5.
FIG. 6 shows a $^1H$ NMR spectrum of carboxymethylated modified starch prepared in Preparation Example 6.

[DETAILED DESCRIPTION OF THE EMBODIMENTS]

The terminology used herein is for the purpose of describing particular embodiments only and is not intended to limit the invention. The singular forms are intended to include the plural forms as well, unless the context clearly indicates otherwise. It will be further understood that the terms "include", "have", or "possess" when used in this specification, specify the presence of stated features, steps, components, or combinations thereof, but do not preclude the presence or addition of one or more other features, steps, components, or combinations thereof.

Also, as used herein, when a layer or an element is mentioned to be formed "on", or "above" layers or elements, the layer or element may be directly formed on the layers or elements, or other layers or elements may be additionally formed between the layers, on a subject, or on a substrate.

As the present invention can be variously modified and have various forms, specific embodiments thereof are shown by way of examples and will be described in detail. However, it is not intended to limit the present invention to the particular form disclosed and it should be understood that the present invention includes all modifications, equivalents, and replacements within the idea and technical scope of the present invention.

In addition, the terminologies are used merely to refer to specific embodiments, and are not intended to restrict the present disclosure. Singular expressions of the present disclosure may include plural expressions unless they are differently expressed contextually.

Meanwhile, the terminology "(meth)acrylate" used herein includes both acrylate and methacrylate.

The terminology "polymer" in the present disclosure is in a state in which a water-soluble ethylene-based unsaturated monomer is polymerized, and may include all moisture content ranges, or all particle diameter ranges. Among the polymers, a polymer having a moisture content of about 40 wt% or more after polymerization and before drying may be referred to as a hydrogel polymer, and particles in which the hydrogel polymer is pulverized and dried may be referred to as a cross-linked polymer.

In addition, the terminology "super absorbent polymer particle" refers to a particulate material containing a cross-linked polymer in which a modified polysaccharide having at least partially neutralized acidic groups is polymerized.

In addition, the terminology "super absorbent polymer" is used to encompass all of a cross-linked polymer in which a modified polysaccharide having at least partially neutralized acidic groups is polymerized or a base resin in the form of powder consisting of super absorbent polymer particles in which the cross-linked polymer is pulverized, and the cross-linked polymer or the base resin further processed, for example, surface cross-linking, fine reassembling, drying, pulverization, classification, etc., to be in a state suitable for commercialization, depending on the context.

The super absorbent polymer that is commonly used in the art is produced by polymerizing an acrylic acid-based monomer together with a cross-linking agent in the presence of a polymerization initiator, but the super absorbent polymer thus prepared does not have biodegradability, which causes environmental problems.

Accordingly, development has been made for super absorbent polymers capable of exhibiting biodegradability, and polysaccharide, polyaspartic acid, and polyglutamic acid have been discussed as biodegradable materials capable of producing such biodegradable resins. However, these have reduced the absorption capacity, which is an important physical property of the super absorbent polymer, and thus there have been difficulties in replacing the super absorbent polymer produced with an acrylic acid-based monomer.

Therefore, the present inventors have found that when a super absorbent polymer is prepared by using a modified polysaccharide having at least one of a maleic acid group (-OCOCH=CHCOOH) and a carboxymethyl group ($-CH_2-COOH$) as a monomer, not only the biodegradability is excellent, but also various physical properties of super absorbent polymers are excellent compared to those of known biodegradable materials, thereby completing the present disclosure.

Hereinafter, a biodegradable super absorbent polymer and a preparation method of the same according to specific embodiments of the present invention will be described in more detail.

**Biodegradable super absorbent polymer**

Specifically, a biodegradable super absorbent polymer according to an embodiment of the present disclosure includes a cross-linked polymer of a monomer containing a modified polysaccharide having at least one of a maleic acid group (-OCOCH=CHCOOH) and a carboxymethyl group ($-CH_2-COOH$).

Herein, the cross-linked polymer has a structure formed by polymerizing a monomer containing a modified polysaccharide having at least a partially neutralized maleic acid group (-OCOCH=CHCOOH) and carboxymethyl group ($-CH_2-COOH$). At this time, the modified polysaccharides are polymerized and grown into a main chain, and at the same time, they are polymerized by the functional groups in the structure to have a cross-linked polymerized structure. Accordingly, various physical properties of the super absorbent polymer can be significantly improved.

In addition, the cross-linked polymer includes the repeating unit derived from modified polysaccharide in an amount of 60 parts by weight or more. That is, it includes a higher content of modified polysaccharide than other unsaturated monomers, and furthermore, the main chain composed of the modified polysaccharide is cross-linked and has excellent biodegradability compared to super absorbent polymers having a high content of other unsaturated monomers. Preferably, the cross-linked polymer may include the modified polysaccharide-derived repeating unit in an amount of 80 parts by weight or more, 85 parts by weight or more, 90 parts by weight or more, or 95 parts by weight or more.

Further, the modified polysaccharide of the present disclosure has a molecular weight of 190,000 g/mol or more, and the super absorbent polymer obtained by cross-linking polymerization of the modified polysaccharide within the corresponding molecular weight has been confirmed to be advantageous in forming a cross-linked structure compared to the super absorbent polymer obtained by polymerization of a modified polysaccharide having a lower molecular weight, thereby exhibiting excellent overall absorption properties.

Meanwhile, the polysaccharide refers to a polymeric carbohydrate molecule composed of glucose repeating units. In this case, the polysaccharide is also referred to including a polymer molecule composed of a glucosamine unit in which an amino group is introduced into a hydroxyl group bonded to the 2nd carbon atom within the glucose repeating unit, and/or an N-acetylglucosamine unit in which an N-acetylamino group is introduced into a hydroxyl group bonded to the 2nd carbon atom within the glucose repeating unit. Therefore, as the polysaccharide, any compound usually known as a polysaccharide can be used without limitation. Examples thereof include, but are not limited to, starch composed of glucose repeating units, chitosan composed of glucosamine repeating units and N-acetylglucosamine repeating units, and the like.

In addition, the modified polysaccharide used in the biodegradable super absorbent polymer is a concept distinguished from unmodified polysaccharides usually obtained naturally or synthetically, and means that a hydroxyl group (-OH) in the glucose repeating unit forming the polysaccharide is substituted with another functional group by chemical treatment and/or heat treatment. Such a modified polysaccharide may exhibit different physical properties from unmodified polysaccharide due to a functional group introduced instead of the hydroxyl group (-OH).

For example, the modified polysaccharide is modified starch, modified cellulose, or modified chitosan.

Meanwhile, at least one of a maleic acid group (-OCOCH=CHCOOH) and a carboxymethyl group ($-CH_2-COOH$) is substituted in the modified polysaccharide, and at least a part of the maleic acid group (-OCOCH=CHCOOH) and the carboxymethyl group ($-CH_2-COOH$) is neutralized.

Further, the maleic acid group (-OCOCH=CHCOOH) in the polysaccharide may be introduced by maleic acid or maleic acid anhydride. For example, a hydroxyl group (-OH) present in an unmodified polysaccharide molecule may be substituted with a maleic acid group (-OCOCH=CHCOOH) by maleic anhydride.

In addition, the carboxymethyl group ($-CH_2-COOH$) in the polysaccharide may be introduced by any one of

glycolic acid, monochloroacetic acid and a salt thereof, and specific examples thereof may include glycolilc acid, monochloroacetic acid, sodium monochloroacetate, sodium glycolate, potassium monochloroacetate, and potassium glycolate. For example, a hydroxyl group (-OH) present in an unmodified polysaccharide molecule may be substituted with a carboxymethyl group ($-CH_2-COOH$) by glycolic acid.

At this time, the degree of substitution (DS) of the maleic acid group (-OCOCH=CHCOOH) of the modified polysaccharide is represented by "$DS_M$" in the following, which ranges from 0.15 to 1.5. When the degree of substitution of the maleic acid group is less than 0.15, a large amount of modified polysaccharides that do not participate in cross-linking polymerization may remain, and when the degree of substitution of the maleic acid group is greater than 0.65, maleic anhydride, which is used in a large amount for introducing a maleic acid group, may not be completely removed, which may affect the physical properties of the final super absorbent polymer. Preferably, the degree of substitution of the maleic acid group is 0.20 or more, 0.25 or more, 0.30 or more, 0.35 or more, 0.40 or more, 0.45 or more, 0.50 or more, 0.55 or more, 0.60 or more, 0.65 or more, 0.70 or more, 0.75 or more, 0.80 or more, or 0.85 or more; and 1.45 or less, 1.40 or less, 1.35 or less, 1.30 or less, 1.25 or less, 1.20 or less, 1.15 or less, 1.10 or less, 1.05 or less, 1.00 or less, or 0.95 or less.

In addition, the degree of substitution (DS) of the carboxymethyl group ($-CH_2-COOH$) of the modified polysaccharide is represented by "DSc" in the following, which ranges from 0.20 to 1.2. When the degree of substitution of the carboxymethyl group is less than 0.20, a large amount of modified polysaccharides that do not participate in cross-linking polymerization may remain, and when the degree of substitution of the carboxymethyl group is greater than 1.2, glycolic acid, which is used in a large amount for introducing a carboxymethyl group, may not be completely removed, which may affect the physical properties of the final super absorbent polymer. Preferably, the degree of substitution of the carboxymethyl group is 0.21 or more, 0.22 or more, 0.23 or more, 0.24 or more, or 0.25 or more; and 1.15 or less, 1.1 or less, 1.05 or less, or 1.0 or less.

Herein, the degree of substitution of the maleic acid group or of carboxymethyl group means the average number of hydroxyl groups (-OH) substituted with maleic acid groups or carboxymethyl groups per glucose repeating unit. That is, since three hydroxyl groups are present per glucose repeating unit, the theoretical maximum degree of substitution is 3. "The degree of substitution is 0.1" means that one hydroxyl group is substituted per 10 glucose repeating units. The degree of substitution of the maleic acid group or carboxymethyl group can be calculated through the $^1H$ NMR analysis of the finally prepared modified polysaccharide. For more detailed contents, refer to Preparation Examples described later.

Meanwhile, a part of the maleic acid groups and carboxymethyl groups of the modified polysaccharide is neutralized, and the degree of neutralization thereof can be adjusted according to the type of the modified polysaccharide and the physical properties of the final super absorbent polymer to be realized. For example, in the case of modified starch, it has high molecular weight and thus allows water insolubility or neutralization degree to increase, so that the carboxyl group (COOH) can be neutralized to the carboxylate ($COO^-$) form, thereby increasing the solubility in water. Specifically, the degree of neutralization of the modified polysaccharide may be 40 to 95 mol %, 40 to 80 mol %, or 45 to 75 mol %.

In one embodiment, the modified polysaccharide may include at least one of the repeating units represented by the following Chemical Formulae 1 to 4:

[Chemical Formula 1]

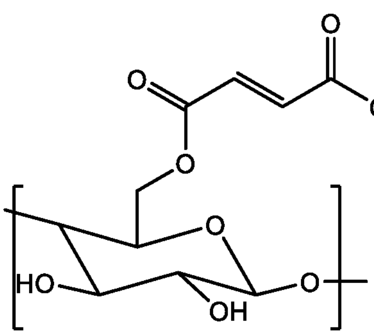

[Chemical Formula 2]

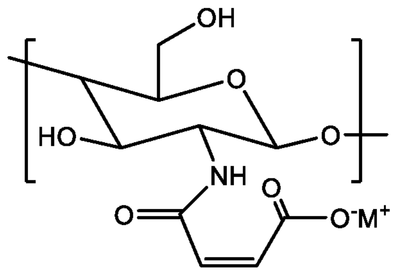

[Chemical Formula 3]

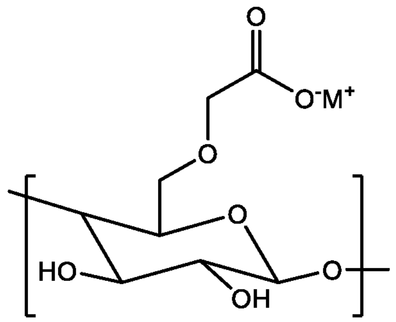

[Chemical Formula 4]

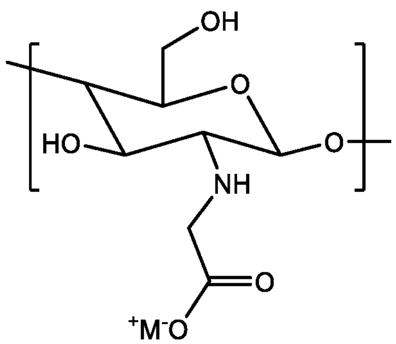

in Chemical Formulae 1 to 4,
M is each independently hydrogen or an alkali metal.

Specifically, in Chemical Formulae 1 to 4, $M^+$ is each independently $H^+$, $Na^+$, or $K^+$.

In one example, when the modified polysaccharide is modified starch or modified dextrin, it may include the repeating units represented by Chemical Formula 1 and Chemical Formula 3, and when the modified polysaccharide is modified chitosan, it may include the repeating units represented by Chemical Formula 2 and Chemical Formula 4.

Further, the modified polysaccharide has a weight average molecular weight of 190,000 g/mol or more. When the weight average molecular weight of the modified starch is too low, there is a problem that sufficient cross-linking cannot be performed and a large amount of unreacted modified starch remains, and when the weight average molecular weight is too high, an entanglement phenomenon of the polymer chains of modified polysaccharides with high molecular weight may occur, which makes acid treatment and functional group introduction itself difficult. Thus, there is a problem that the production of a modified polysaccharide is not easy. Preferably, the modified polysaccharide has a weight average molecular weight of 200,000 g/mol or more, 250,000 g/mol or more, or 300,000 g/mol or more, and 1,000,000,000 g/mol or less, 500,000,000 g/mol or less, 100,000,000 g/mol or less, or 50,000,000 g/mol or less.

The suitable molecular weight of the modified polysaccharide may vary depending on the type of material. Specifically, the molecular weight of the modified starch is 400,000 g/mol to 1,000,000,000 g/mol. Preferably, the molecular weight of the modified starch is 1,000,000 g/mol or more, 5,000,000 g/mol or more, 10,000,000 g/mol or more, or 20,000,000 g/mol or more, and 500,000,000 g/mol or less, 100,000,000 g/mol or less, or 50,000,000 g/mol or less.

In addition, the molecular weight of the modified cellulose or modified chitosan may be 190,000 g/mol to 10,000,000 g/mol. Preferably, it may be 200,000 g/mol or more, 250,000 g/mol or more, or 300,000 g/mol or more, and 5,000,000 g/mol or less, 2,000,000 g/mol or less, 1,000,000 g/mol or less, or 700,000 g/mol or less.

Herein, the weight average molecular weight (Mw) can be measured using gel permeation chromatography (GPC/MALLS). A specific method for measuring the weight average molecular weight is described in detail in Examples to be described later.

Meanwhile, the modified polysaccharide may be modified starch. The modified starch may include amylose and amylopectin in a weight ratio of 1:99 to 50:50 based on the total weight. Modification in which the hydroxyl group bonded to the 6th carbon of the glucose repeating unit is substituted with another substituent may occur in both amylose and amylopectin; However, from the viewpoint of processability and solubility, it is advantageous that the amylopectin content is equal to or higher than the amylose content.

Further, the modified starch may have a gelatinization temperature of 50 to 90 °C, and a peak viscosity (BU) of 50 to 1,000.

The modified starch may be a starch in which at least one starch selected from the group consisting of potato starch, corn starch, rice starch, wheat starch, tapioca starch and sweet potato starch has been modified. In particular, potato starch having a high content of amylopectin may be preferable from the viewpoint of processability and solubility.

Meanwhile, the monomer may further include an acrylic acid-based compound having an acidic group of which at least a part is neutralized.

The acrylic acid-based monomer is a compound represented by the following Chemical Formula 3:

[Chemical Formula 3] R-COOM'

in Chemical Formula 2,

R is a C2 to C5 alkyl group containing an unsaturated bond, and
M' is a hydrogen atom, a monovalent or divalent metal, an ammonium group, or an organic amine salt.

Preferably, the monomer may be one or more selected from the group consisting of (meth)acrylic acid, and a monovalent (alkali) metal salt, a divalent metal salt, an ammonium salt and an organic amine salt thereof.

When (meth)acrylic acid and/or a salt thereof is used as the acrylic acid-based monomer, a super absorbent polymer having improved absorbency can be obtained.

Herein, the acrylic acid-based monomer has acidic groups, wherein at least a part of the acidic groups may be neutralized. Preferably, those partially neutralized with an alkali substance such as sodium hydroxide, potassium hydroxide, ammonium hydroxide or the like may be used. In this case, the acrylic acid-based monomer may have a degree of neutralization in the range of 40 to 95 mol%, 40 to 80 mol%, or 45 to 75 mol%. The range of the degree of neutralization may vary depending on the final physical properties. An excessively high degree of neutralization causes the neutralized monomers to be precipitated, and thus polymerization may not readily occur. On the contrary, an excessively low degree of neutralization not only deteriorates absorbency of the polymer, but also gives the polymer hard-to-handle properties, such as those of an elastic rubber.

In addition, the cross-linked polymer of the monomers including the modified polysaccharide may be cross-linked and polymerized by an internal cross-linking agent. The term 'internal cross-linking agent' as used herein is a term used to distinguish it from a surface cross-linking agent commonly used for cross-linking the surface of super absorbent polymer particles, and serves to cross-link and polymerize the above-mentioned modified polysaccharide. The cross-linking in the above step proceeds both on the surface and on the inside, but when the surface cross-linking process of the super absorbent polymer particles proceeds, the surface of the particles of the finally prepared super absorbent polymer has a structure cross-linked by a surface cross-linking agent, and the inside of the particles has a structure cross-linked by the internal cross-linking agent.

When a monomer including a modified polysaccharide is subjected to a cross-linking polymerization in the presence of an internal cross-linking agent, the cross-linked polymer has a three-dimensional network structure in which the main chains formed by polymerizing polysaccharides are additionally cross-linked by the internal cross-linking agent. When the cross-linked polymer has the three-dimensional network structure cross-linked by the internal cross-linking agent in this way, the centrifuge retention capacity and absorbency under pressure, which are various physical properties of the super absorbent polymer, can be significantly improved compared to the case not additionally cross-linked by an internal cross-linking agent.

As the internal cross-linking agent, any compound can be used as long as it enables the introduction of cross-linking during polymerization of the modified polysaccharide. Non-limiting examples of the internal cross-linking agent

may include multifunctional cross-linking agents, such as N,N'-methylenebisacrylamide, trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol(meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin, or ethylene carbonate, which can be used alone or in combination of two or more thereof, but are not limited thereto. Preferably, N,N'-methylenebisacrylamide or polyethylene glycol diacrylate may be used.

Cross-linking polymerization of the modified starch in the presence of such an internal cross-linking agent may be performed by thermal polymerization, photopolymerization or hybrid polymerization in the presence of a polymerization initiator, optionally a thickener, plasticizer, storage stabilizer, antioxidant, and the like, and detailed contents thereof will be described later.

Further, the super absorbent polymer may be in the form of particles having an average particle diameter of 150 to 850 μm. In this case, the particle size can be measured according to EDANA (European Disposables and Nonwovens Association) WSP 220.3. More specifically, in the super absorbent polymer composition, about 90 wt%, preferably 95 wt% or more, based on the total weight, may be super absorbent polymer particles having a particle diameter of about 150 to 850 μm, and less than about 10 wt%, more specifically less than about 5 wt%, may be fine powders having a particle diameter of less than about 150 μm. When the super absorbent polymer contains a large amount of fine powders having a particle diameter of less than 150 μm, it may reduce various physical properties of the super absorbent polymer, which is thus not preferable.

Further, the biodegradable super absorbent polymer may have a centrifuge retention capacity (CRC) of 20 to 50 g/g as measured according to EDANA WSP 241.3.

**Preparation method of biodegradable super absorbent polymer**

Meanwhile, the biodegradable super absorbent polymer may be prepared by the following preparation method including the steps of:

preparing a monomer composition including a modified polysaccharide having at least one of a maleic acid group (-OCOCH=CHCOOH) and a carboxymethyl group ($-CH_2-COOH$) in the presence of a polymerization initiator;
preparing a hydrogel polymer by performing cross-linking polymerization on the monomer composition; and
drying, pulverizing, and classifying the hydrogel polymer,
wherein at least a part of the maleic acid group and the carboxymethyl group is neutralized,
the cross-linked polymer contains 60 parts by weight or more of a modified polysaccharide-derived repeating unit, and a molecular weight of the modified polysaccharide is 190,000 g/mol or more.

Meanwhile, the modified polysaccharide may be prepared by reacting the polysaccharide with at least one of maleic acid, maleic acid anhydride, glycolic acid, monochloroacetic acid and a salt thereof.

Specifically, a modified polysaccharide may be prepared by including the step of reacting the polysaccharide with at least one of maleic acid, maleic acid anhydride, glycolic acid, monochloroacetic acid and a salt thereof to prepare a polysaccharide into which at least one of a maleic acid group and a carboxymethyl group is introduced. Further, the reaction of the polysaccharide with at least one of maleic acid, maleic acid anhydride, glycolic acid, monochloroacetic acid and a salt thereof can proceed at a temperature of 50 to 100 °C for 4 to 12 hours.

In one example, the acid-treated polysaccharide can be maleated using maleic anhydride, whereby a maleic acid group-substituted polysaccharide can be prepared.

In addition, before preparing the modified polysaccharide, the unmodified polysaccharide may be acid-treated. Such acid treatment destroys the rigid structure of starch by hydrogen bonding, and thus proceeds in order to increase the production efficiency for modification of polysaccharides such as maleation and carboxylmetilation. Specifically, the acid treatment can be performed using an acidic solution such as hydrochloric acid at a temperature of 25 to 50 °C for 6 to 48 hours.

Subsequently, a step of forming a hydrogel polymer by performing cross-linking polymerization on the prepared modified starch in the presence of a polymerization initiator may be performed.

The step may include a step of preparing a monomer composition by mixing the modified starch and a polymerization initiator, and a step of forming a hydrogel polymer by performing thermal polymerization or photopolymerization on the monomer composition.

Furthermore, the monomer composition may further include an acrylic acid-based compound having an acidic group of which at least a part is neutralized. In the monomer composition, the modified polysaccharide and the acrylic acid-based compound may be included in a weight ratio of 99:1 to 60:40. When the above-mentioned range is satisfied,

the biodegradable super absorbent polymer can exhibit excellent biodegradability, and at the same time have improved absorbency and water retention capacity.

In addition, the monomer composition may further include an internal cross-linking agent. For details on the internal cross-linking agent, refer to the above. In the monomer composition, the internal cross-linking agent may be used in an amount of 0.1 to 5 parts by weight based on 100 parts by weight of the modified polysaccharide. For example, the internal cross-linking agent may be used in an amount of 0.1 parts by weight or more, or 0.2 parts by weight or more, and 5 parts by weight or less, 3 parts by weight or less, 2 parts by weight or less, 1 part by weight or less, or 0.5 parts by weight or less, based on 100 parts by weight of the modified polysaccharide. When the content of the internal cross-linking agent is too low, cross-linking does not occur sufficiently, and it may be difficult to achieve strength above an appropriate level. When the content of the internal cross-linking agent is too high, the internal cross-linking density may increase, which may make it difficult to achieve a desired water retention capacity.

Further, the polymerization initiator may be appropriately selected depending on the polymerization method. In the case of a thermal polymerization method, a thermal polymerization initiator is used, in the case of a photopolymerization method, a photopolymerization initiator is used, and in the case of a hybrid polymerization method (a method using both heat and light), both a thermal polymerization initiator and a photopolymerization initiator can be used. However, even in the case of performing the photopolymerization method, a certain amount of heat is generated by light irradiation such as ultraviolet irradiation. Further, a certain amount of heat may be generated with the progress of the polymerization reaction, which is an exothermic reaction, a thermal polymerization initiator can be further used.

As the photopolymerization initiator, a compound capable of forming radicals by a light such as UV can be used without limitations in the constitution.

For example, the photopolymerization initiator may be one or more compounds selected from the group consisting of benzoin ether, dialkyl acetophenone, hydroxyl alkylketone, phenyl glyoxylate, benzyl dimethyl ketal, acyl phosphine, and $\alpha$-aminoketone. Meanwhile, specific examples of the acyl phosphine include diphenyl(2,4,6-trimethylbenzoyl)phosphine oxide, phenylbis(2,4,6-trimethylbenzoyl)phosphine oxide, ethyl(2,4,6-trimethylbenzoyl)phenylphosphinate, and the like. More various photopolymerization initiators are well disclosed in "UV Coatings: Basics, Recent Developments and New Application (Elsevier, 2007)" written by Reinhold Schwalm, p 115, and the present disclosure is not limited thereto.

In addition, one or more initiators selected from the group consisting of a persulfate-based initiator, an azo-based initiator, hydrogen peroxide, and ascorbic acid may be used as the thermal polymerization initiator. Specifically, sodium persulfate ($Na_2S_2O_8$), potassium persulfate ($K_2S_2O_8$), ammonium persulfate ($(NH_4)_2S_2O_8$), and the like may be used as examples of the persulfate-based initiators; and 2,2-azobis(2-amidinopropane) dihydrochloride, 2,2-azobis-(N,N-dimethylene)isobutyramidine dihydrochloride, 2-(carbamoylazo)isobutylonitril, 2,2-azobis[2-(2-imidazolin-2-yl)propane] dihydrochloride, 4,4-azobis-(4-cyanovaleric acid), and the like may be used as examples of the azo-based initiators. More various thermal polymerization initiators are well disclosed in 'Principle of Polymerization (Wiley, 1981)' written by Odian, p 203, and the present disclosure is not limited thereto.

The polymerization initiator may be used at a concentration of 2 parts by weight or less based on 100 parts by weight of the modified polysaccharide. That is, when the concentration of the polymerization initiator is too low, the polymerization rate may become slow and thus a large amount of residual monomer may be extracted from the final product, which is thus not preferable. On the contrary, when the concentration of the polymerization initiator is too high, a polymer chain forming a network may become short, and thus, the physical properties of polymer may be degraded, such as increase in the content of water-soluble components and decrease in absorbency under pressure, which is not preferable.

The monomer composition may further include additives such as a thickener, a plasticizer, a storage stabilizer, and an antioxidant, if necessary.

Further, the monomer composition containing the monomer may be in a suspension state when it is insoluble like starch having a high molecular weight, or may be in a solution state dissolved in a solvent such as water when it is water-soluble like dextrin having a low molecular weight. The solid content in the monomer composition, that is, the concentration of the monomer, the internal cross-linking agent and the polymerization initiator can be appropriately adjusted in consideration of the polymerization time, the reaction conditions, and the like. For example, the solid content in the monomer composition may be 10 to 80 wt%, 15 to 60 wt%, or 30 to 50 wt%.

When the monomer composition has the solid content within the above range, it is not necessary to remove unreacted monomers after polymerization by using the gel effect phenomenon that appears in the polymerization reaction of a high-concentration aqueous solution, and at the same time, it may be advantageous to adjust the pulverization efficiency at the time of pulverization of the polymer to be described later.

Herein, any solvent may be used without limitations in the constitution, as long as it is able to dissolve the above-mentioned components. For example, the solvent may include one or more selected from water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone,

cyclohexanone, cyclopentanone, diethylene glycol monomethyl ether, diethylene glycol ethylether, toluene, xylene, butyrolactone, carbitol, methyl cellosolve acetate, and N,N-dimethylacetamide

Meanwhile, the cross-linking polymerization of the modified polysaccharide can proceed without particular limitation, as long as the hydrogel polymer can be formed by thermal polymerization, photopolymerization, or hybrid polymerization.

Specifically, the polymerization method is largely divided into the thermal polymerization and the photopolymerization according to an energy source of the polymerization. In the case of thermal polymerization, it is generally carried out in a reactor equipped with an agitation spindle, such as a kneader. In the case of photopolymerization, it may be carried out in a reactor equipped with a movable conveyor belt or in a container with a flat bottom. However, the polymerization method is just an example, and the present disclosure is not limited thereto.

For example, in the reactor equipped with an agitation spindle such as a kneader, the hydrogel polymer obtained by thermal polymerization by supplying hot air or heating the reactor may be discharged to a reactor outlet in the form of several centimeters to several millimeters depending on a shape of the agitation spindle provided in the reactor. Specifically, a size of the hydrogel polymer obtained may vary depending on the concentration and injection rate of the monomer mixture to be injected, and a hydrogel polymer having a weight average particle diameter of 2 to 50 mm may be usually obtained.

In addition, when photopolymerization is performed in the reactor equipped with a movable conveyor belt or in a container with a flat bottom as described above, a hydrogel polymer in the form of a sheet having a belt width may usually be obtained. At this time, a thickness of the polymer sheet may vary depending on the concentration and injection rate or amount of the monomer composition to be injected, and it is preferable to supply the monomer composition so that the polymer in the form of a sheet has a thickness of 0.5 to 5 cm. When the monomer composition is supplied to such an extent that the thickness of the polymer sheet is too thin, the production efficiency may be low. When the thickness of the polymer sheet exceeds 5 cm, the polymerization reaction may not occur evenly over the entire thickness due to the excessively thick thickness.

In this case, the hydrogel polymer obtained by the above method may have a moisture content of 40 to 70 wt%. For example, the moisture content of the hydrogel polymer may be 40 wt% or more, 45 wt% or more, or 50 wt% or more, and 70 wt% or less, 65 wt% or less, or 60 wt% or less. When the moisture content of the hydrogel polymer is too low, it becomes difficult to secure an appropriate surface area in the subsequent pulverization step, and the drying efficiency may decrease. When the moisture content of the hydrogel polymer is too high, the pressure received in the subsequent pulverization step may increase, so that the absorbency under pressure may be lowered, which may require a lot of energy and a long time in the drying step after pulverization.

Meanwhile, "moisture content" in the present disclosure is the content of moisture in the entire weight of the hydrogel polymer, and it means a value of which the weight of the dried polymer is subtracted from the weight of the hydrogel polymer. Specifically, the moisture content is defined as a value calculated from the weight loss due to moisture evaporation from the polymer in the process of increasing the temperature of the polymer in a crumb state and drying the same through infrared heating. At this time, the drying for measuring the moisture content is performed by raising the temperature from room temperature to about 50 °C and then vacuum drying at 50 °C for about 6 hours.

Meanwhile, after the preparation of the hydrogel polymer, a coarse pulverization step of pulverizing the prepared hydrogel polymer can be optionally performed before the subsequent drying and pulverization steps.

The coarse pulverization step is a step for increasing drying efficiency in a subsequent drying step and controlling the particle size of the super absorbent polymer powder to be finally prepared. At this time, a pulverizing machine used herein may include, but its configuration is not limited to, any one selected from the group consisting of a vertical pulverizer, a turbo cutter, a turbo grinder, a rotary cutter mill, a cutter mill, a disc mill, a shred crusher, a crusher, a meat chopper, and a disc cutter. However, the present disclosure is not limited thereto.

In the coarse pulverization step, the hydrogel polymer may be pulverized to have a diameter of about 2 to 10 mm. It is technically difficult to pulverize the hydrogel polymer to have a diameter of less than 2 mm because of its high moisture content, and there may be a phenomenon that the pulverized particles cohere with each other. Meanwhile, when the polymer is pulverized to have a diameter of larger than 10 mm, the efficiency enhancing effect in the subsequent drying step may be insignificant.

Next, a step of drying, pulverizing, and classifying the hydrogel polymer prepared in the above step is performed to obtain a base resin.

The drying method may be selected and used without limitation in the constitution if it is a method commonly used in the art. Specifically, the drying step may be performed by a method such as hot air provision, infrared radiation, microwave radiation, UV ray radiation, or the like.

Specifically, the drying can be performed under vacuum conditions at a temperature of less than 100 °C, specifically, at a temperature of about 30 °C to about 80 °C. When the drying temperature is 100 °C or higher, the modified polysaccharide can be decomposed, which is thus not suitable. When the drying temperature is less than 30 °C, the drying time may be too long.

Meanwhile, the drying may be performed for about 20 to about 90 minutes, in consideration of the process efficiency and the like, but it is not limited thereto.

After the drying step, the moisture content of the polymer may be about 5 to about 10 wt%.

After the drying step, a pulverization step is performed.

The pulverization step can be performed so that the particle diameter of the polymer powder, that is, the base resin, is about 150 to about 850 μm. In order to pulverize the polymer into such diameter, a pin mill, a hammer mill, a screw mill, a roll mill, a disc mill, or a jog mill may be used as the pulverizing machine, but the present disclosure is not limited thereto.

Further, in order to control physical properties of the super absorbent polymer to be finally prepared after the pulverization step as described above, a step of classifying the pulverized polymer powder according to the particle diameter may be further performed.

Preferably, a polymer having a particle diameter of about 150 to about 850 μm is classified, and only the polymer powder having such a particle diameter can be used as a base resin to undergo a surface cross-linking reaction, which can be finally commercialized.

The base resin obtained as a result of the above process may have a powder form including an acrylic acid-based monomer and a cross-linked polymer cross-linked by an internal cross-linking agent. Specifically, the base resin may have a powder form having a particle diameter of 150 to 850 μm.

Meanwhile, there is further provided a hygiene product including the above-described biodegradable super absorbent polymer.

Hereinafter, preferred embodiments are presented to facilitate the understanding of the invention. However, the following examples are for illustrative purposes only and are not intended to limit the present disclosure.

**Preparation Example 1: Preparation of modified chitosan (M/S)**

20 g of chitosan (200 - 600 mPa.s, 0.5% in 0.5% acetic acid at 20 °C, manufactured by TCI) and 100 g of maleic anhydride were added to a 500 ml three-necked flask containing 200 ml of water, and then stirred at 70 °C for 6 hours. After the reaction, the mixture was precipitated in acetone solvent and filtered to remove excess maleic anhydride, and dried at 50 °C for 24 hours or more to obtain maleated chitosan.

The weight average molecular weight of the prepared chitosan was $3.3*10^5$ g/mol, and the degree of substitution ($DS_M$) of maleic acid group (-OCOCH=CHCOOH) was 0.94. At this time, the degree of substitution of the substituent group was calculated based on the integral ratio of the peak corresponding to vinyl (CH=CH, 6.3 ppm, 5.8 ppm) of the maleic acid group in $^1$H NMR, and the specific calculation method is as follows.

First, a $^1$H NMR spectrum of the prepared maleated chitosan was obtained, which is shown in FIG. 1. Referring to FIG. 1, it can be confirmed that a maleic acid group has been introduced into chitosan, as the peaks appeared at 6.3 ppm, 5.8 ppm corresponding to the vinyl group of the maleic acid group. Based on the integral ratio of these peaks, it was possible to confirm the degree of substitution of the maleic acid group (-OCOCH=CHCOOH) of the maleated chitosan.

**Preparation Example 2: Preparation of modified starch 1 (M/S-1)**

20 g of starch (Starch from potato, manufactured by Aldrich) and 100 g of maleic anhydride were added to a 250 ml three-necked flask containing 200 ml of water, and then stirred at 70 °C for 6 hours. After the reaction, the mixture was precipitated in acetone solvent and filtered to remove excess maleic anhydride, and dried at 50 °C for 24 hours or more to obtain maleated modified starch.

The weight average molecular weight of the prepared modified starch was $3.4*10^7$ g/mol, and the degree of substitution ($DS_M$) of maleic acid group (-OCOCH=CHCOOH) was 0.88.

At this time, the degree of substitution of the substituent group was calculated based on the integral ratio of the peaks corresponding to vinyl (CH=CH, 6.45ppm, 6.28ppm) of the maleic acid group in the spectrum after obtaining $^1$H NMR spectrum of the prepared maleated modified starch, which is shown in FIG. 2. For details on the specific calculation method, refer to the above method for calculating the degree of substitution of the modified chitosan (M/S) in Preparation Example 1, and the following Preparation Examples were also calculated in the same manner.

**Preparation Example 3: Preparation of modified starch 2 (M/S-2)**

Starch (Starch from poatato, manufactured by Aldrich), isopropyl alcohol (IPA), and NaOH aqueous solution (concentration: about 40%) were mixed in a 500 mL RBF (Round Bottom Flask), and stirred at room temperature for about 20 minutes. A weight ratio of the mixture was 1:4:0.8 (starch:IPA:NaOH aqueous solution). Subsequently, sodium monochloroacetate was added in an amount of about 80 parts by weight based on 100 parts by weight of the starch, heated to 60 °C, stirred for about 1 hour, and then cooled to room temperature. After cooling to room temperature, the

solvent was removed. Then, the reactant was dissolved in water, precipitated in methanol to remove water-soluble impurities, filtered to recover starch, and dried in a vacuum drying oven at 40 °C for about 12 hours to obtain a solid target product.

50 mg of the obtained solid target product and 200 mg of a 30% $D_2SO_4$ in $D_2O$ solution were mixed, and stirred at 50 °C for about 1 hour to induce a hydrolysis reaction, followed by $^1H$ NMR analysis. The $^1H$ NMR spectrum of the prepared carboxymethylated modified starch is shown in FIG. 3.

The weight average molecular weight of the prepared modified starch was $1.2*10^7$ g/mol, and the degree of substitution (DSc) of carboxymethyl group was 0.6.

**Preparation Example 4: Preparation of modified starch 3 (M/S-3)**

Starch (Starch from corn, manufactured by Samyang), isopropyl alcohol (IPA), and NaOH aqueous solution (concentration: about 40%) were mixed in a 500 mL RBF (Round Bottom Flask), and stirred at room temperature for about 20 minutes. A weight ratio of the mixture was 1:4:0.8 (starch:IPA:NaOH aqueous solution). Subsequently, sodium monochloroacetate was added in an amount of about 80 parts by weight based on 100 parts by weight of the starch, heated to 60 °C, stirred for about 1 hour, and then cooled to room temperature. After cooling to room temperature, the solvent was removed. Then, the reactant was dissolved in water, precipitated in methanol to remove water-soluble impurities, filtered to recover starch, and dried in a vacuum drying oven at 40 °C for about 12 hours to obtain a solid target product.

50 mg of the obtained solid target product and 200 mg of a 30% $D_2SO_4$ in $D_2O$ solution were mixed, and stirred at 50 °C for about 1 hour to induce a hydrolysis reaction, followed by $^1H$ NMR analysis. The $^1H$ NMR spectrum of the prepared carboxymethylated modified starch is shown in FIG. 4.

The weight average molecular weight of the prepared modified starch was $3*10^7$ g/mol, and the degree of substitution (DSc) of carboxymethyl group was 0.6.

Preparation Example 5: Preparation of modified starch 4 (M/S-4)

Starch (Starch from poatato, manufactured by Daesang), isopropyl alcohol (IPA), and NaOH aqueous solution (concentration: about 40%) were mixed in a 500 mL RBF (Round Bottom Flask), and stirred at room temperature for about 20 minutes. A weight ratio of the mixture was 1:4:0.8 (starch:IPA:NaOH aqueous solution). Subsequently, sodium monochloroacetate was added in an amount of about 80 parts by weight based on 100 parts by weight of the starch, heated to 60 °C, stirred for about 1 hour, and then cooled to room temperature. After cooling to room temperature, the solvent was removed. Then, the reactant was dissolved in water, precipitated in methanol to remove water-soluble impurities, filtered to recover starch, and dried in a vacuum drying oven at 40 °C for about 12 hours to obtain a solid target product.

50 mg of the obtained solid target product and 200 mg of a 30% $D_2SO_4$ in $D_2O$ solution were mixed, and stirred at 50 °C for about 1 hour to induce a hydrolysis reaction, followed by $^1H$ NMR analysis. The $^1H$ NMR spectrum of the prepared carboxymethylated modified starch is shown in FIG. 5.

The weight average molecular weight of the prepared modified starch was $1.9*10^7$ g/mol, and the degree of substitution (DSc) of carboxymethyl group was 0.6.

**Preparation Example 6: Preparation of modified starch 5 (M/S-5)**

Starch (Starch from corn, manufactured by Daesang), isopropyl alcohol (IPA), and NaOH aqueous solution (concentration: about 40%) were mixed in a 500 mL RBF (Round Bottom Flask), and stirred at room temperature for about 20 minutes. A weight ratio of the mixture was 1:4:0.8 (starch:IPA:NaOH aqueous solution). Subsequently, sodium monochloroacetate was added in an amount of about 80 parts by weight based on 100 parts by weight of the starch, heated to 60 °C, stirred for about 1 hour, and then cooled to room temperature. After cooling to room temperature, the solvent was removed. Then, the reactant was dissolved in water, precipitated in methanol to remove water-soluble impurities, filtered to recover starch, and dried in a vacuum drying oven at 40 °C for about 12 hours to obtain a solid target product.

50 mg of the obtained solid target product and 200 mg of a 30% $D_2SO_4$ in $D_2O$ solution were mixed, and stirred at 50 °C for about 1 hour to induce a hydrolysis reaction, followed by $^1H$ NMR analysis. The $^1H$ NMR spectrum of the prepared carboxymethylated modified starch is shown in FIG. 6.

The weight average molecular weight of the prepared modified starch was $2.4*10^7$ g/mol, and the degree of substitution (DSc) of carboxymethyl group was 0.8.

**Comparative Preparation Example** 1: **Preparation of modified starch (M)**

20 g of starch (Starch soluble, manufactured by Samchun chemicals) and 60 g of maleic anhydride were added to a 500 ml three-necked flask containing 200 ml of water, and then stirred at 70 °C for 6 hours. After the reaction, the mixture was precipitated in acetone solvent and filtered to remove excess maleic anhydride, and dried at 50 °C for 24 hours or more to obtain maleated modified starch (M).

The weight average molecular weight of the finally prepared modified starch (M) was $1.5*10^5$ g/mol, and the degree of substitution ($DS_M$) of maleic acid group (-OCOCH=CHCOOH) was 0.81.

**Comparative Preparation Example 2: Preparation of modified chitosan (M-1)**

20 g of chitosan (5 - 20 mPa.s, 0.5% in 0.5% acetic acid at 20 °C, manufactured by TCI) and 100 g of maleic anhydride were added to a 500 ml three-necked flask containing 200 ml of water, and then stirred at 70 °C for 6 hours. After the reaction, the mixture was precipitated in acetone solvent and filtered to remove excess maleic anhydride, and dried at 50 °C for 24 hours or more to obtain maleated modified starch (M).

The weight average molecular weight of the finally prepared modified chitosan (M-1) was $5*10^4$ g/mol, and the degree of substitution ($DS_M$) of maleic acid group (-OCOCH=CHCOOH) was 1.11.

**Comparative Preparation Example 3: Preparation of modified starch (M-2)**

Starch (Starch soluble, manufactured by Duksan pharmaceutical industrial co.), isopropyl alcohol (IPA), and NaOH aqueous solution (concentration: about 40%) were mixed in a 500 mL RBF (Round Bottom Flask), and stirred at room temperature for about 20 minutes. A weight ratio of the mixture was 1:4:0.8 (starch:IPA:NaOH aqueous solution). Subsequently, sodium monochloroacetate was added in an amount of about 80 parts by weight based on 100 parts by weight of the starch, heated to 60 °C, stirred for about 1 hour, and then cooled to room temperature. After cooling to room temperature, the solvent was removed. Then, the reactant was dissolved in water, precipitated in methanol to remove water-soluble impurities, filtered to recover starch, and dried in a vacuum drying oven at 40 °C for about 12 hours to obtain a solid target product.

The weight average molecular weight of the finally prepared modified starch (M-2) was $9.2*10^4$ g/mol, and the degree of substitution ($DS_M$) of carboxymethyl group was 0.68.

**Example 1**

50 mL of distilled water and 3 g of modified chitosan (M/S) of Preparation Example 1 were added to a 250 mL RBF (Round Bottom Flask), and the mixture was stirred in an oil bath at 35 °C for 30 minutes or more to sufficiently dissolve the modified chitosan in distilled water. Thereafter, an initiator was added. As the initiator, about 0.03 g of ammonium persulfate was added. After adding the initiator, cross-linking was performed by further stirring at a temperature of about 70 °C for about 4 hours. After cross-linking, ethanol was added to precipitate a polymer material containing the cross-linked polymer, followed by filtration, and drying in a vacuum drying oven at 40 °C overnight to obtain a target polymer material. The dried polymer obtained in this way was pulverized using a pulverizing machine and classified with an ASTM standard mesh to obtain a base resin having a particle diameter of 300 to 600 μm, which was referred to as a super absorbent polymer.

**Example 2**

A super absorbent polymer was prepared in the same manner as in Example 1, except that the modified starch (M/S-1) of Preparation Example 2 was used instead of the modified chitosan (M/S) in Example 1.

**Example 3**

A super absorbent polymer was prepared in the same manner as in Example 1, except that the modified starch (M/S-2) of Preparation Example 3 was used instead of the modified chitosan (M/S) in Example 1.

**Example 4**

A super absorbent polymer was prepared in the same manner as in Example 1, except that the modified starch (M/S-3) of Preparation Example 4 was used instead of the modified chitosan (M/S) in Example 1.

**Example 5**

A super absorbent polymer was prepared in the same manner as in Example 1, except that the modified starch (M/S-4) of Preparation Example 5 was used instead of the modified chitosan (M/S) in Example 1.

**Example 6**

A super absorbent polymer was prepared in the same manner as in Example 1, except that the modified starch (M/S-5) of Preparation Example 6 was used instead of the modified chitosan (M/S) in Example 1.

**Comparative Example 1**

A super absorbent polymer was prepared in the same manner as in Example 1, except that the modified starch (M) of Comparative Preparation Example 1 was used instead of the modified chitosan (M/S) in Example 1.

**Comparative Example 2**

A super absorbent polymer was prepared in the same manner as in Example 1, except that the modified chitosan (M-1) of Comparative Preparation Example 2 was used instead of the modified chitosan (M/S) in Example 1.

**Comparative Example 3**

A super absorbent polymer was prepared in the same manner as in Example 1, except that the modified starch (M-2) of Comparative Preparation Example 3 was used instead of the modified chitosan (M/S) in Example 1.

**Experimental Examples**

The physical properties of the super absorbent polymers prepared in Examples and Comparative Examples were evaluated by the following method, and the results are shown in Table 1 below.

Unless otherwise specified, all of the following physical property evaluations were performed at constant temperature and humidity (23±1°C, relative humidity 50±10%). The physiological saline or saline means 0.9 wt% sodium chloride (NaCl) aqueous solution.

(1) Weight average molecular weight

The weight average molecular weight was measured by GPC/MALLS, and the detailed measurement method is as follows.

1) Preparation of mobile phase

1000 mL of 100 mM NaNOs aqueous solution containing 0.02% $NaN_3$ was filtered through a solvent clarification system.

2) Preparation of sample solution

5 mL of 100 mM NaNOs aqueous solution containing 0.02% $NaN_3$ was added to 25 mg of the sample, heated at 110 °C for 1 hour, and filtered through a 0.45 μm nylon syringe filter for analysis.

3) GPC/MALLS conditions

stationary phase: Shodex OH-Pak 804 컬럼, Shodex OH-Pak 80 컬럼
mobile phase: 100 mM NaNOs aqueous solution containing 0.02% $NaN_3$
flow rate: 0.4 mL/min
stationary phase temperature: 25 °C
injection volume: 100 microliters
analysis time: 120 minutes

(2) Centrifuge Retention Capacity (CRC)

The centrifugal retention capacity of each polymer by water absorption capacity under a non-loading condition was measured according to the EDANA WSP 241.3.

After inserting Wo (g, about 0.2 g) of the super absorbent polymer uniformly in a nonwoven fabric envelope and sealing the same, it was soaked in saline (0.9 wt%) at room temperature. After 30 minutes, the envelope was centrifuged at 250G for 3 minutes to drain, and the weight $W_2$ (g) of the envelope was measured. Further, after carrying out the same operation without using the polymer, the weight $W_1$ (g) of the envelope was measured. Then, CRC (g/g) was calculated by using the obtained weight values according to the following Equation.

[Equation 1]

$$CRC\ (g/g) = \{[W_2(g) - W_1(g)]/W_0(g)\} - 1$$

(3) Biodegradability Test

It was measured according to KS M ISO 14851 standard.

[Table 1]

| | Modified polysaccharide | Mw of modified polysaccharide (g/mol) | CRC (g/g) | Biodegradability (%) |
|---|---|---|---|---|
| Example 1 | modified chitosan (M/S) | $3.3*10^5$ | 32.8 | 83 |
| Example 2 | modified starch (M/S-1) | $3.4*10^7$ | 24.2 | 99 |
| Example 3 | modified starch (M/S-2) | $1.2*10^7$ | 24.2 | 78 |
| Example 4 | modified starch (M/S-3) | $3*10^7$ | 31.2 | 70 |
| Example 5 | modified starch (M/S-4) | $1.9*10^7$ | 37.4 | 66 |
| Example 6 | modified starch (M/S-5) | $2.4*10^7$ | 32.4 | 71 |
| Comparative Example 1 | modified starch (M) | $1.5*10^5$ | 13.2 | 85 |
| Comparative Example 2 | modified chitosan (M-1) | $5.0*10^4$ | 5.7 | 95 |
| Comparative Example 3 | modified starch (M-2) | $9.2*10^4$ | 13.2 | 74 |

Referring to Table 1, it was confirmed that the biodegradable super absorbent polymer containing the modified polysaccharide of high molecular weight according to the present disclosure had excellent biodegradability with excellent water absorption properties.

## Claims

1. A biodegradable super absorbent polymer comprising a cross-linked polymer of a monomer containing a modified polysaccharide having at least one of a maleic acid group (-OCOCH=CHCOOH) and a carboxymethyl group ($-CH_2-COOH$),

   wherein at least a part of the maleic acid group and the carboxymethyl group is neutralized,

the cross-linked polymer contains 60 parts by weight or more of a modified polysaccharide-derived repeating unit, and
a molecular weight of the modified polysaccharide is 190,000 g/mol or more.

**2.** The biodegradable super absorbent polymer of Claim 1,
wherein a degree of substitution of the maleic acid group in the modified polysaccharide is 0.15 to 1.5.

**3.** The biodegradable super absorbent polymer of Claim 1,
wherein a degree of substitution of the carboxymethyl group in the modified polysaccharide is 0.2 to 1.2.

**4.** The biodegradable super absorbent polymer of Claim 1,
wherein the modified polysaccharide is modified starch, modified cellulose, or modified chitosan.

**5.** The biodegradable super absorbent polymer of Claim 1,
wherein the maleic acid group is introduced by maleic acid or maleic acid anhydride.

**6.** The biodegradable super absorbent polymer of Claim 1,
wherein the carboxymethyl group is introduced by any one of glycolic acid, monochloroacetic acid and a salt thereof.

**7.** The biodegradable super absorbent polymer of Claim 1,
wherein the modified polysaccharide comprises at least one of the repeating units represented by the following Chemical Formulae 1 to 4:

[Chemical Formula 1]

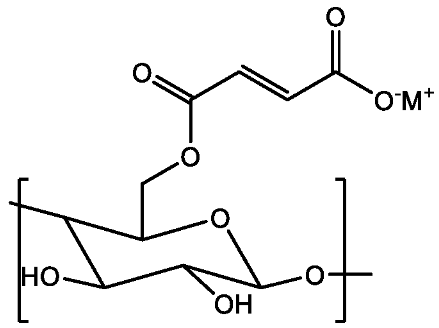

[Chemical Formula 2]

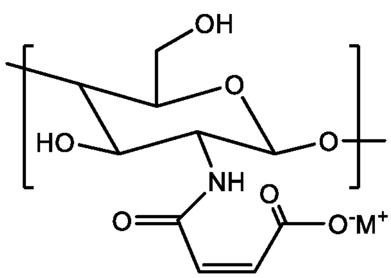

[Chemical Formula 3]

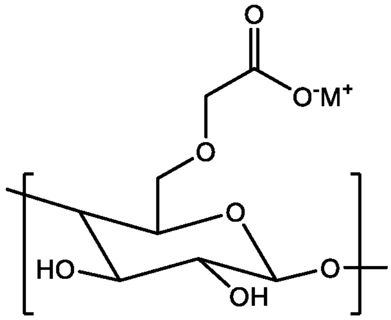

[Chemical Formula 4]

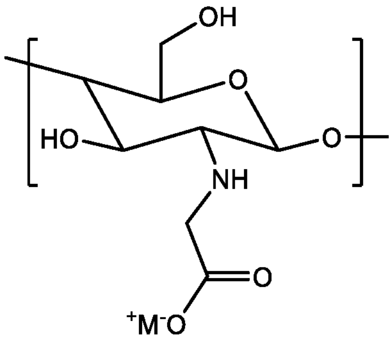

in Chemical Formulae 1 to 4,
M is each independently hydrogen or an alkali metal.

**8.** The biodegradable super absorbent polymer of Claim 1, wherein the cross-linked polymer comprises 80 parts by weight or more of a modified polysaccharide-derived repeating unit.

**9.** The biodegradable super absorbent polymer of Claim 1, wherein a molecular weight of the modified polysaccharide is 300,000 g/mol to 1,000,000,000 g/mol.

**10.** The biodegradable super absorbent polymer of Claim 1, wherein a molecular weight of the modified polysaccharide is 300,000 g/mol to 50,000,000 g/mol.

**11.** The biodegradable super absorbent polymer of Claim 4, wherein a molecular weight of the modified starch is 400,000 g/mol to 1,000,000,000 g/mol.

**12.** The biodegradable super absorbent polymer of Claim 4, wherein a molecular weight of the modified cellulose or modified chitosan is 190,000 g/mol to 10,000,000 g/mol.

**13.** The biodegradable super absorbent polymer of Claim 1, wherein the monomer further comprises an acrylic acid-based compound having at least partially neutralized acidic groups.

**14.** The biodegradable super absorbent polymer of Claim 1, wherein the cross-linked polymer is cross-linked by an internal cross-linking agent.

**15.** A preparation method of a biodegradable super absorbent polymer comprising the steps of:

preparing a monomer composition comprising a modified polysaccharide having at least one of a maleic acid

group (-OCOCH=CHCOOH) and a carboxymethyl group ($-CH_2-COOH$) in the presence of a polymerization initiator;
preparing a hydrogel polymer by performing cross-linking polymerization on the monomer composition; and drying, pulverizing, and classifying the hydrogel polymer,
wherein at least a part of the maleic acid group and the carboxymethyl group is neutralized,
the cross-linked polymer contains 60 parts by weight or more of a modified polysaccharide-derived repeating unit, and
a molecular weight of the modified polysaccharide is 190,000 g/mol or more.

**16.** The preparation method of a biodegradable super absorbent polymer of Claim 15,
wherein the modified polysaccharide is prepared by reacting the polysaccharide with at least one of maleic acid, maleic acid anhydride, glycolic acid, monochloroacetic acid and a salt thereof.

**17.** The preparation method of a biodegradable super absorbent polymer of Claim 15,
wherein the monomer composition further comprises an internal cross-linking agent.

**18.** A hygiene product comprising the biodegradable super absorbent polymer according to any one of Claims 1 to 14.

【FIG. 1】

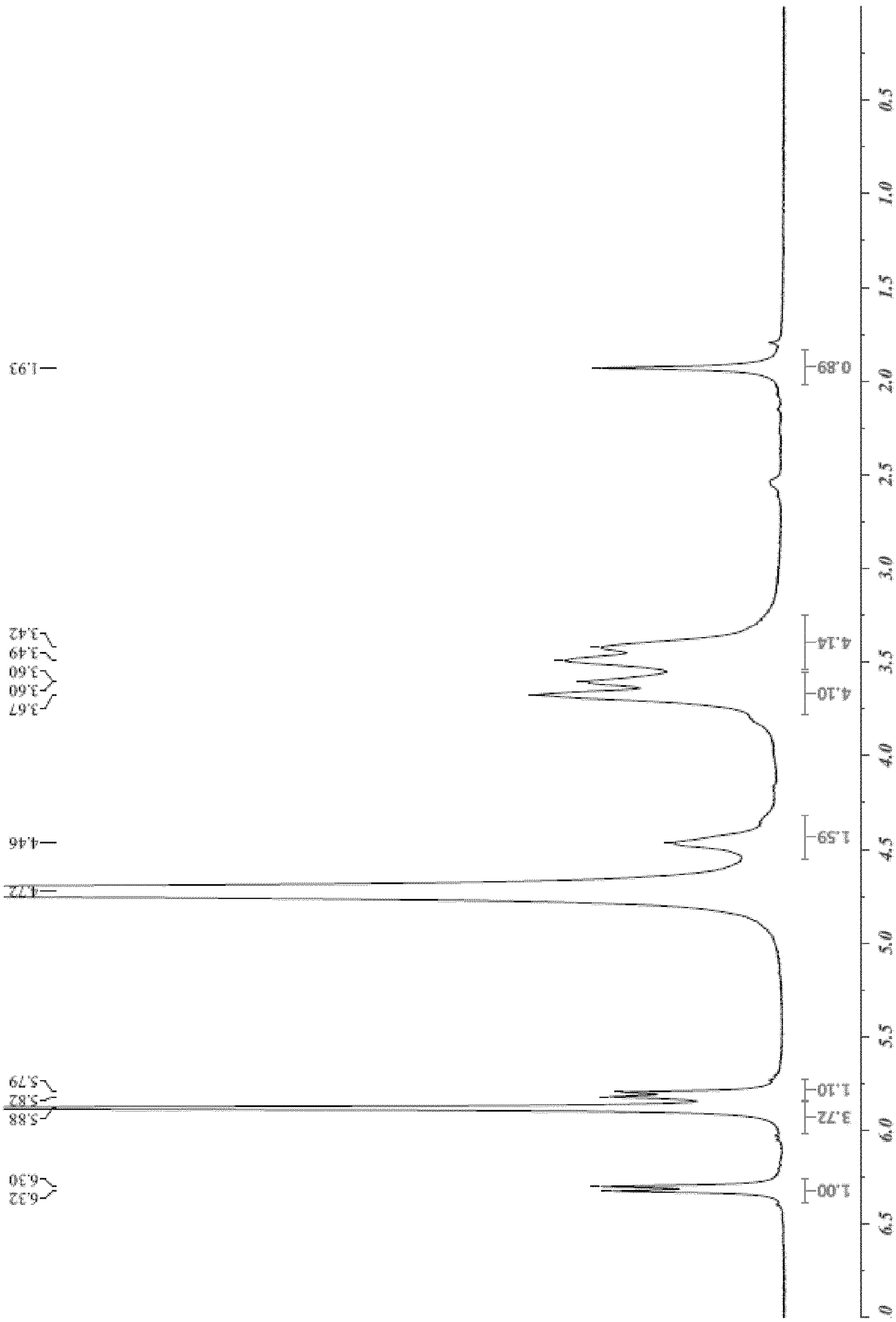
1.93
3.42
3.49
3.60
3.60
3.67
4.46
4.72
5.79
5.82
5.88
6.30
6.32
0.89
4.14
4.10
1.59
1.10
3.72
1.00
0.5
1.0
1.5
2.0
2.5
3.0
3.5
4.0
4.5
5.0
5.5
6.0
6.5

【FIG. 2】

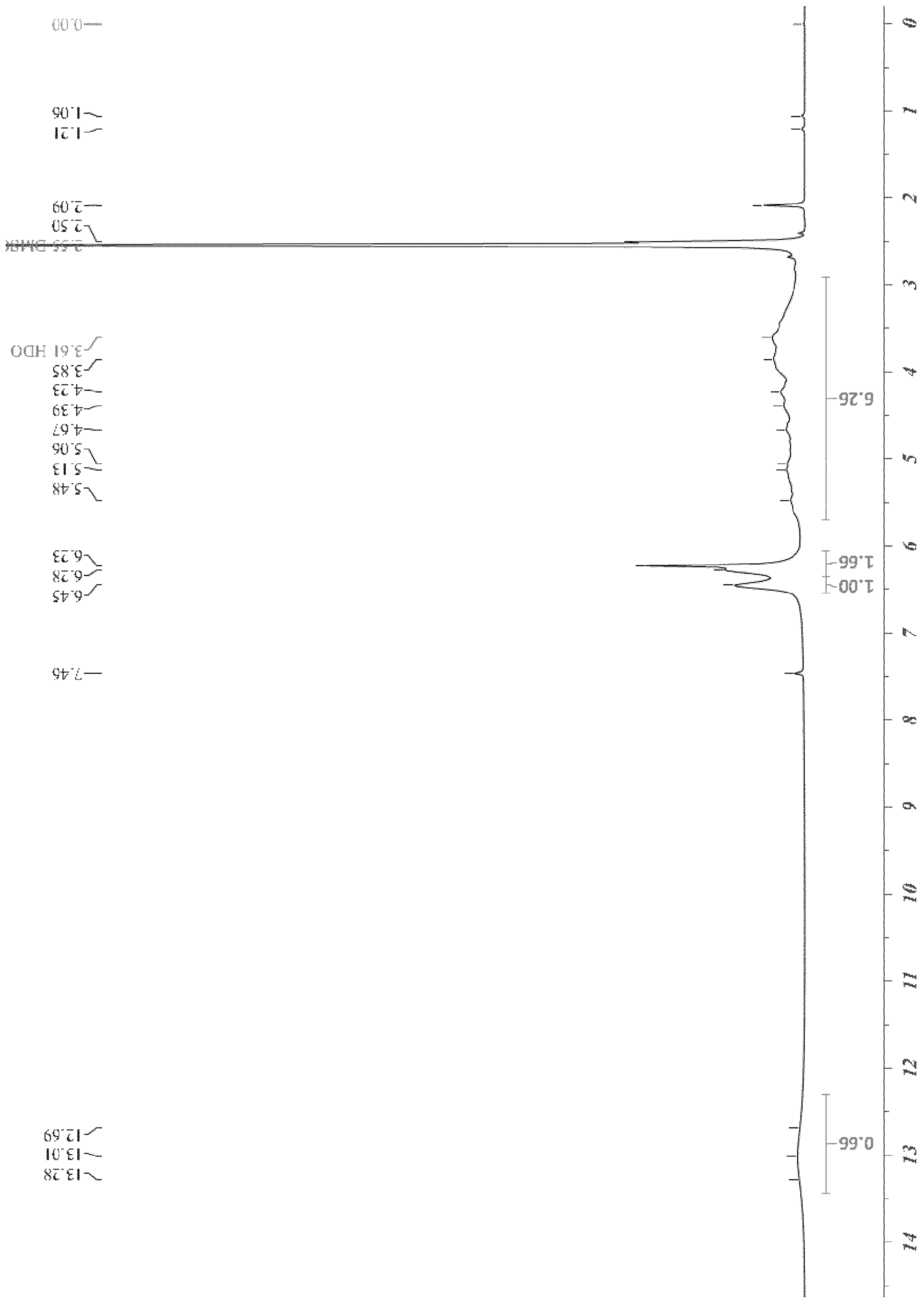
0.00
1.06
1.21
2.09
2.50
3.61 HDO
3.85
4.23
4.39
4.67
5.06
5.13
5.48
6.23
6.28
6.45
7.46
12.69
13.01
13.28
6.26
1.66
1.00
0.66
0
1
2
3
4
5
6
7
8
9
10
11
12
13
14

【FIG. 3】

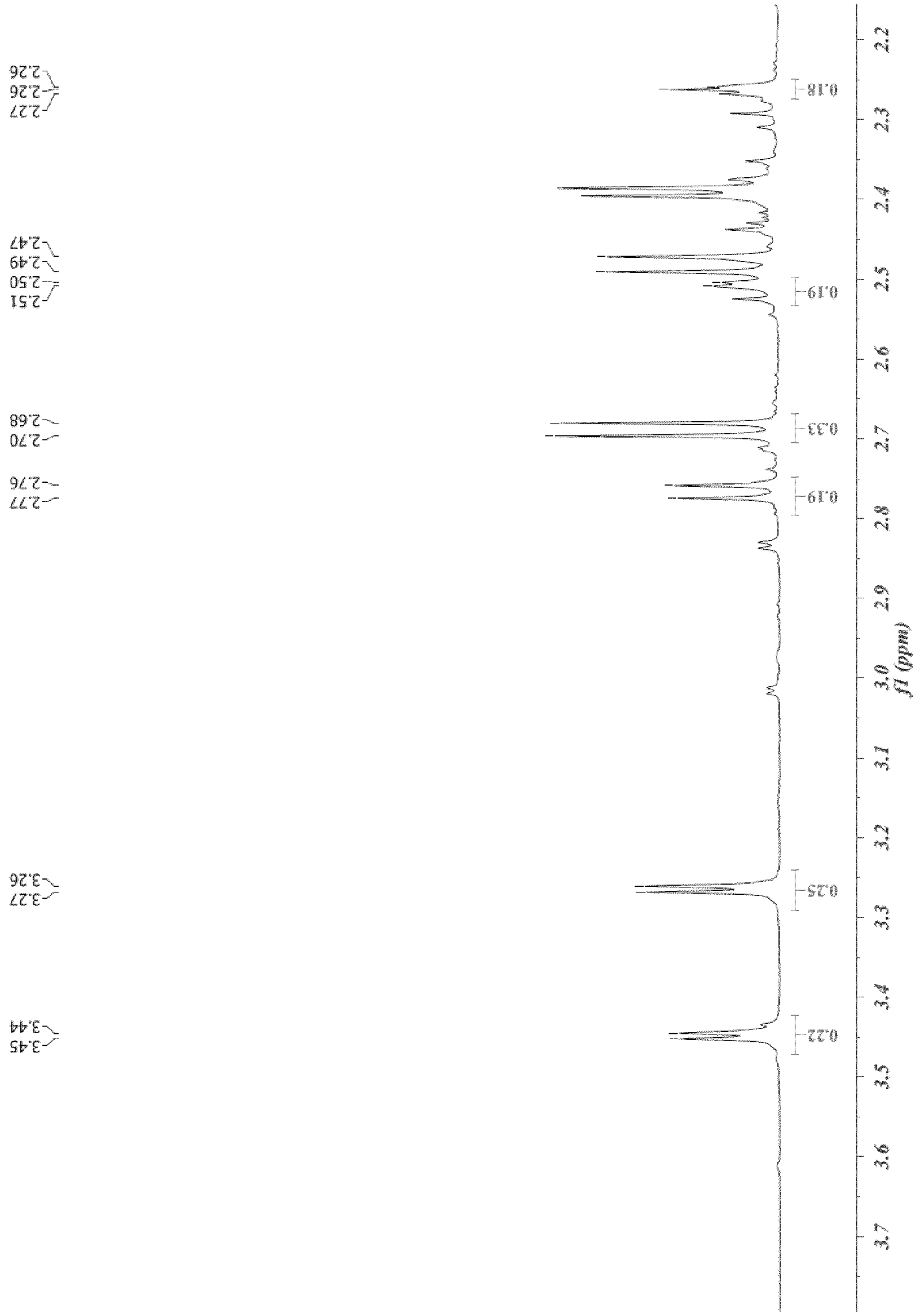
2.26
2.26
2.27
2.47
2.49
2.50
2.51
2.68
2.70
2.76
2.77
3.26
3.27
3.44
3.45
0.18
0.19
0.33
0.19
0.25
0.22
2.2
2.3
2.4
2.5
2.6
2.7
2.8
2.9
3.0
3.1
3.2
3.3
3.4
3.5
3.6
3.7
f1 (ppm)

【FIG. 4】

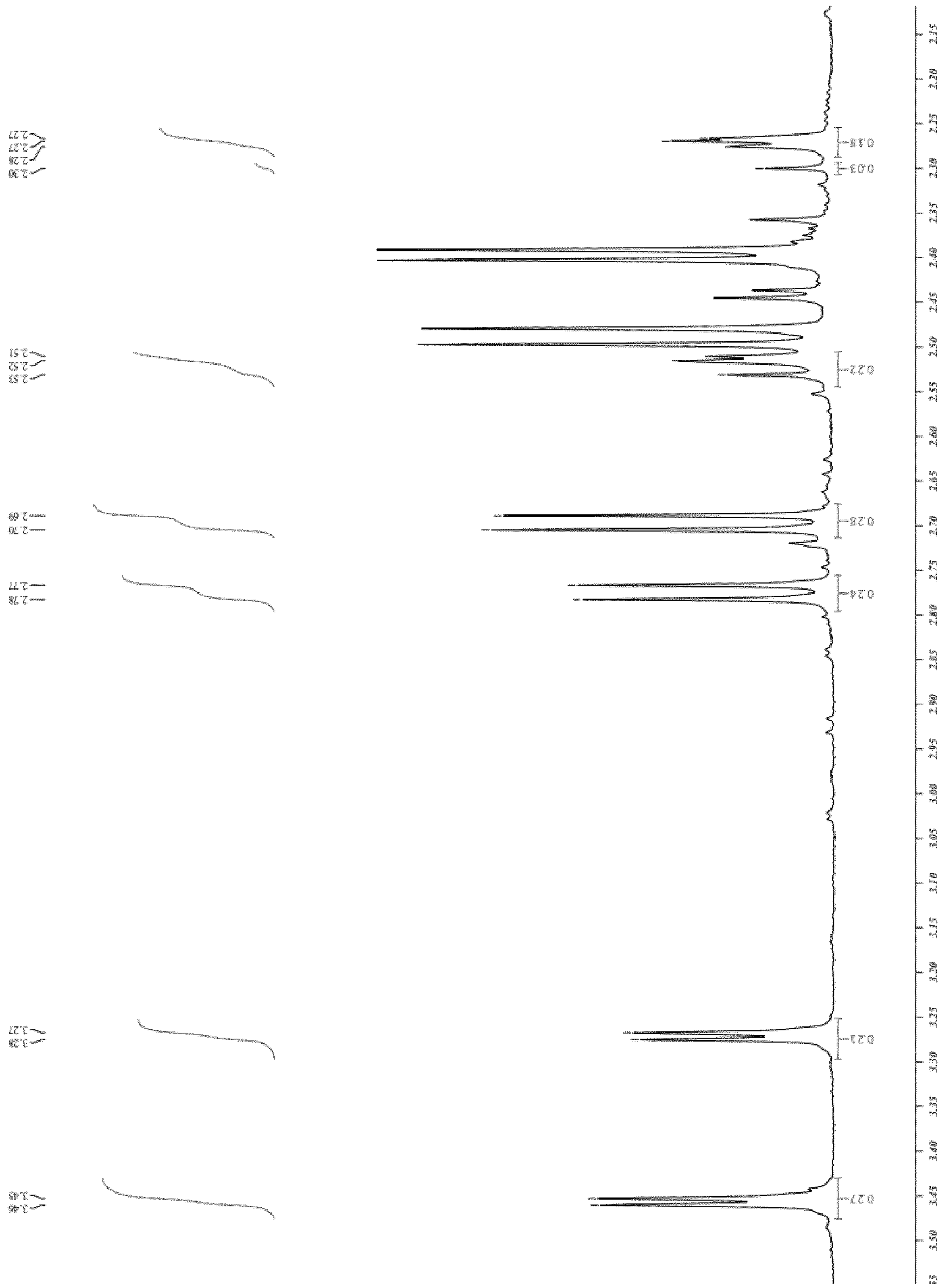

【FIG. 5】

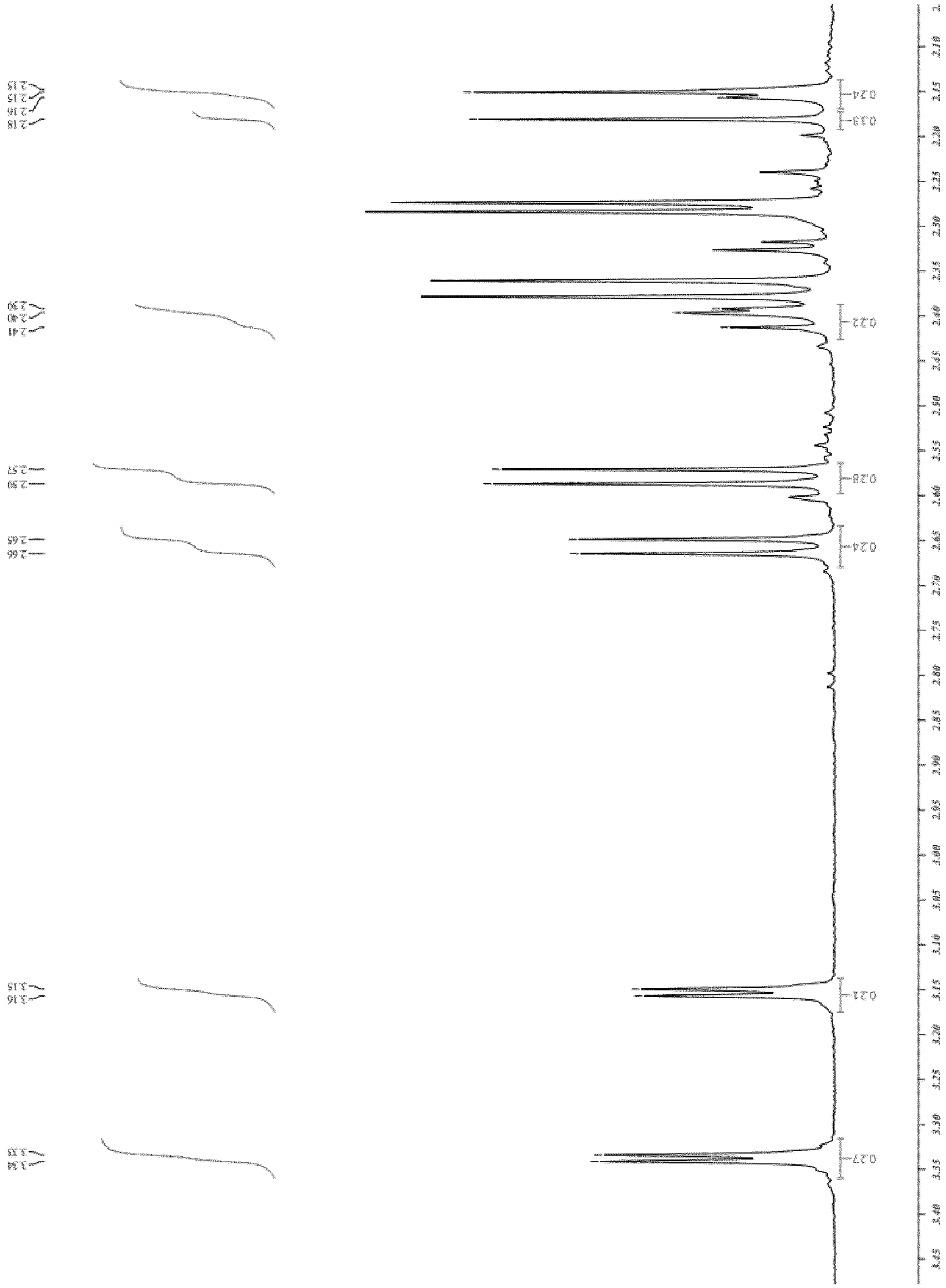

【FIG. 6】

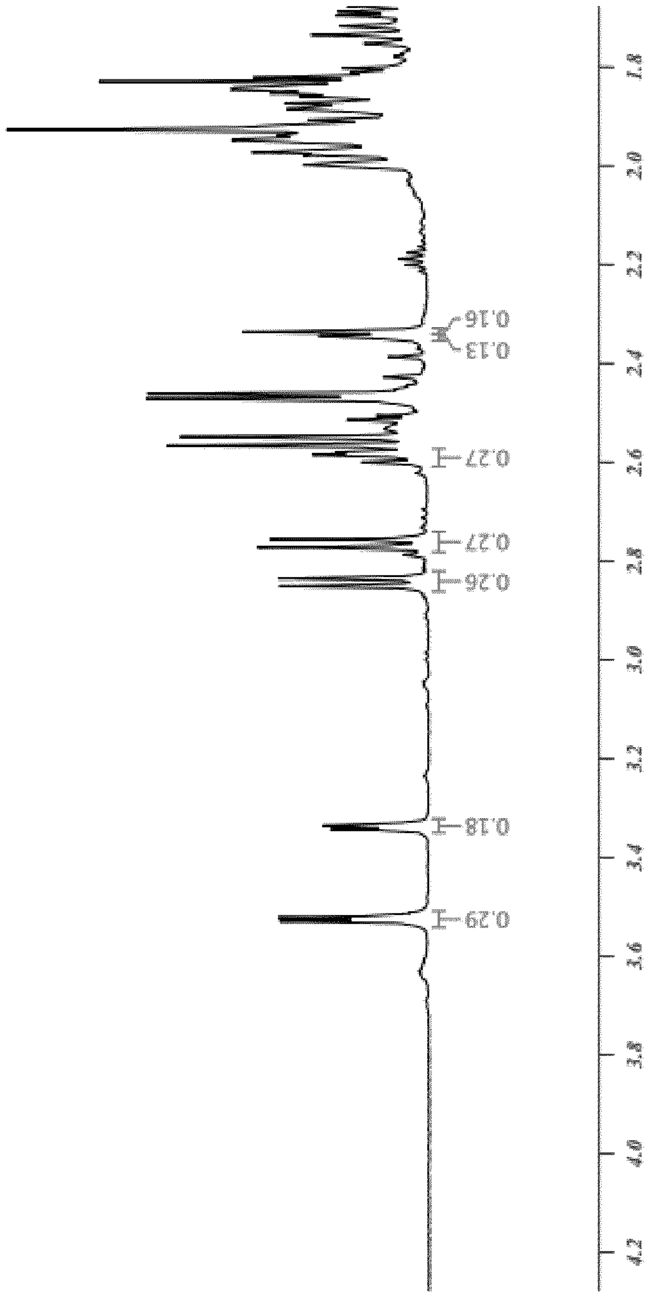

## INTERNATIONAL SEARCH REPORT

International application No.
**PCT/KR2023/008444**

**A. CLASSIFICATION OF SUBJECT MATTER**

**C08L 5/08**(2006.01)i; **C08L 1/28**(2006.01)i; **C08L 3/04**(2006.01)i; **C08B 37/08**(2006.01)i; **C08B 31/04**(2006.01)i; **C08B 11/12**(2006.01)i; **C08J 3/24**(2006.01)i

According to International Patent Classification (IPC) or to both national classification and IPC

**B. FIELDS SEARCHED**

Minimum documentation searched (classification system followed by classification symbols)

C08L 5/08(2006.01); A61L 15/60(2006.01); C08F 220/06(2006.01); C08F 251/00(2006.01); C08F 290/10(2006.01); C08G 73/10(2006.01); C08L 51/08(2006.01); C08L 67/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 말레산기(maleic acid group), 카복시메틸기(carboxymethyl group), 변성 폴리사카라이드(modified polysaccharide), 가교 중합체(crosslinked polymer), 생분해성 고흡수성 수지(biodegradable superabsorbent resin)

**C. DOCUMENTS CONSIDERED TO BE RELEVANT**

| Category* | Citation of document, with indication, where appropriate, of the relevant passages | Relevant to claim No. |
|---|---|---|
| X<br>Y | KR 10-2022-0041752 A (LG CHEM, LTD.) 01 April 2022 (2022-04-01)<br>See claims 1-3, 6-9 and 12; and paragraphs [0060]-[0074], [0097] and [0225]. | 1,2,4,5,7-18<br>3,6 |
| Y | BIDGOLI, H. et al. Effect of carboxymethylation conditions on the water-binding capacity of chitosan-based superabsorbents. Carbohydrate research. 2010, vol. 345, pp. 2683-2689.<br>See abstract; and page 2683. | 3,6 |
| A | CN 104558442 A (QINGDAO WENCHUANG TECHNOLOGY CO., LTD.) 29 April 2015 (2015-04-29)<br>See entire document. | 1-18 |
| A | KR 10-2001-0082336 A (MITSUI CHEMICALS, INC.) 29 August 2001 (2001-08-29)<br>See entire document. | 1-18 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 September 2023** | **15 September 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208**<br>Facsimile No. **+82-42-481-8578** | Telephone No. |

Form PCT/ISA/210 (second sheet) (July 2022)

**INTERNATIONAL SEARCH REPORT**

International application No.
**PCT/KR2023/008444**

**C. DOCUMENTS CONSIDERED TO BE RELEVANT**

| Category* | Citation of document, with indication, where appropriate, of the relevant passages | Relevant to claim No. |
|---|---|---|
| A | CN 107325502 A (SHAANXI UNIVERSITY OF SCIENCE AND TECHNOLOGY) 07 November 2017 (2017-11-07)<br>See entire document. | 1-18 |

Form PCT/ISA/210 (second sheet) (July 2022)

**INTERNATIONAL SEARCH REPORT**
**Information on patent family members**

International application No.
**PCT/KR2023/008444**

| Patent document cited in search report | Publication date (day/month/year) | Patent family member(s) | Publication date (day/month/year) |
|---|---|---|---|
| KR 10-2022-0041752 A | 01 April 2022 | CN 114901714 A | 12 August 2022 |
| | | EP 4063413 A1 | 28 September 2022 |
| | | JP 2023-509980 A | 10 March 2023 |
| | | US 2023-0087087 A1 | 23 March 2023 |
| | | WO 2022-065843 A1 | 31 March 2022 |
| CN 104558442 A | 29 April 2015 | None | |
| KR 10-2001-0082336 A | 29 August 2001 | CN 100332765 A | 23 January 2002 |
| | | EP 1142925 A1 | 10 October 2001 |
| | | EP 1142925 A4 | 09 October 2002 |
| | | JP 2000-239378 A | 05 September 2000 |
| | | WO 00-39194 A1 | 06 July 2000 |
| CN 107325502 A | 07 November 2017 | CN 107325502 B | 26 March 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020220078925
- KR 1020230076830


### Non-patent literature cited in the description


- UV Coatings: Basics, Recent Developments and New Application. Elsevier, 2007, 115
- Principle of Polymerization. Wiley, 1981, 203